(19) Europäisches Patentamt
European Patent Office
Office européen des brevets

(11) Veröffentlichungsnummer: **0 107 023**
**B1**

(12) # EUROPÄISCHE PATENTSCHRIFT

(45) Veröffentlichungstag der Patentschrift: **17.12.86**

(51) Int. Cl.⁴: **A 01 N 25/04**, A 61 K 9/10

(21) Anmeldenummer: **83109164.0**

(22) Anmeldetag: **16.09.83**

(54) **Homogene wässrige Formulierungen.**

(30) Priorität: **30.09.82 DE 3236240**

(43) Veröffentlichungstag der Anmeldung:
**02.05.84 Patentblatt 84/18**

(45) Bekanntmachung des Hinweises auf die Patenterteilung:
**17.12.86 Patentblatt 86/51**

(84) Benannte Vertragsstaaten:
**AT BE CH DE FR GB IT LI NL**

(56) Entgegenhaltungen:
**EP-A-0 055 401**
**EP-A-0 062 181**
**CH-C-275 705**
**DE-A-2 041 480**
**DE-B-1 121 814**
**DE-C-824 949**
**FR-A-2 187 226**
**FR-A-2 452 250**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Wirth, Wolfgang, Dr., Hönscheidstrasse 4 E, D-5202 Hennef 1 (DE)**
Erfinder: **Hausmann, Heinz, Dr., Dierath 5, D-5653 Leichlingen 1 (DE)**
Erfinder: **Horstmann, Heinz- Otto, Dr., Löhe 42, D-5060 Bergisch- Gladbach 1 (DE)**
Erfinder: **Niessen, Heinz Josef, Dr., Pannenberg 73, D-5060 Bergisch- Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 107 023 B1

LIBER, STOCKHOLM 1986

**Beschreibung**

Die vorliegende Erfindung betrifft neue, homogene Formulierungen von in Wasser wenig löslichen, festen Wirkstoffen aus der Gruppe der agrochemischen Wirkstoffe, der Wirkstoffe zur Bekämpfung von Schädlingen im Haushalts- und Hygiene-Bereich und/oder der pharmakoligisch wirksamen Stoffe. Die Erfindung betrifft außerdem ein Verfahren zur Herstellung dieser homogenen wäßrigen Formulierungen sowie deren Verwendung.

Es sind bereits wäßrige Emulsionen von zahlreichen in Wasser wenig löslichen agrochemischen Wirkstoffen bekannt, welche neben den Wirkstoffen jeweils noch entweder einen oberflächenaktiven Stoff und ein Verdickungsmittel oder aber eine relativ große Menge an oberflächenaktiven Stoffen enthalten (vgl. DE-A 3009 944, FR-A 2 452 250, DE-A-30 11 611 und JP-A 122 625-77). Dieser Zusatz an Verdickungsmitteln bzw. an großen Tensidmengen ist mit zusätzlichen Kosten verbunden und stellt somit einen gravierenden Nachteil der bekannten wäßrigen Emulsionen dar. Hinzu kommt, daß die bisher beschriebene Herstellung von derartigen Emulsionen nicht allgemein anwendbar ist. Im wesentlichen lassen sich danach nämlich nur solche in Wasser wenig löslichen Wirkstoffe emulgieren, die bei Raumtemperatur flüssig sind oder zumindest einen sehr niedrigen Schmelzpunkt aufweisen. Nachteilig ist auch, daß die bekannten wäßrigen Emulsionen häufig nicht ausreichend kältestabil sind, und daß in manchen Fällen eine Zwangsemulgierung mit Hilfe von Homogenisatoren erforderlich ist.

Weiterhin sind bereits mit Wasser dispergierbare Konzentrate von pestiziden Wrikstoffen bekannt geworden, die neben dem jeweiligen Wirkstoff lediglich einen oder mehrere Emulgatoren sowie gegebenenfalls eine geringe Menge an organischen Solventien enthalten (vgl. US-A 3 683 075). Ein wesentlicher Nachteil dieser Formulierungen besteht darin, daß vor allem bei niedrigen Wirkstoffgehalten relativ große Mengen an Emulgator eingesetzt werden müssen. Darüber hinaus ist die Viskosität derartiger Konzentrate verhältnismäßig hoch, so daß eine genaue Dosierung schwierig ist.

Außerdem sind aus der DE-C-524 949, der DE-B-1 121 514, der CH-A-275 705 und der EP-A-0 055 401 zahlreiche Alkylaryl-polyglykolether und deren Verwendung als Emulgatoren in agrochemischen Mitteln bekannt. Kombinationen dieser nicht-ionischen Emulgitoren mit ionischen Komponenten sowie mit Cyclohexinon werden in den genannten Druckschriften allerdings nicht erwähnt.

Ferner geht aus der DE-A 3 041 450 hervor, daß Emulgator-Kombinationen aus einer ionischen und einer nicht-ionischen Komponente zur Herstellung von Emulsionen geeignet sind, welche flüssige agrochemische Wirkstoffe, wie zum Beispiel Phosphorsäureester, enthalten. Die betreffenden Emulsionen sind jedoch nur relativ kurze Zeit beständig und enthalten erhebliche Mengen in organischen Solventien.

In der FR-A 3 157 326 werden Mikroemulsionen beschrieben, in denen außer dem Wirkstoff noch einer oder mehrere Emulgatoren und ein Hydrotrop (= Lösungsvermittler), wie unter anderem Cyclohexanon, vorhanden sind. Spezielle Kombinationen aus nichtionischen Emulgatoren im Gemisch mit 4-(n-Dodecyl)-phenyl-sulfonsäure-(2-hydroxyethyl)-ammoniumsalz und Cyclohexanon werden allerdings nicht offenbart.

Schließlich werden in der vorgängigen, aber nicht vorveröffentlichen EP-A 0 063 151 Öl-in-Wasser-Emulsionen beschrieben, die neben dem jeweiligen Wirkstoff eine nichtionische und eine ionische Emulgatorkomponente aufweisen. Der zusätzliche Einsatz von Cyclohexanon in den betreffenden Formulierungen wird jedoch nicht erwähnt.

Es wurden nun neue, homogene wäßrige Formulierungen gefunden, die 0,1 bis 30 Gew.-X mindestens eines nicht hydrolysierenden, mit Cyclohexanon nicht reagierenden, in Wasser bei 20°C zu maximal 0,5 Grw.-% löslichen festen Wirkstoffes aus der Gruppe der agrochemischen Wirkstoffs, der Wirkstoffe zur Bekämpfung von Schädlingen im Haushalts- und Hygiens-Bereich und/oder der pharmakologisch wirksamen Stoffe,

1 bis 15 Gew.-% mindestens eines Alkylaryl-polyglykolethers der Formel

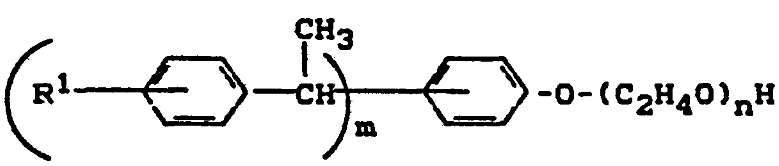 (I)

in welcher
$R^1$ für Alkyl mit 1 bis 12 Kohlenstoffatomen steht,
m für Zahlen von 2 oder 3 steht und
n für Zahlen von 12 bis 40 steht,
im Gemisch mit Alkylarylsulfonsäure-Salz der Formel

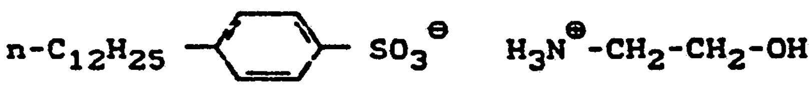

(II)

- 0,5 bis 35 Gew.-X Cyclohexanon
- sowie Wasser und
- gegebenenfalls 0,1 bis 10 Gew.-X an Zusatzstoffen, enthalten, wobei die Summe der Komponenten jeweils 100 Gew.-% beträgt.

Weiterhin wurde gefunden, daß sich die erfindungsgemäßen homogenen wäßrigen Formulierungen dadurch herstellen lassen, daß man

- mindestens einen nicht hydrolysierenden, mit Cyclohexanon nicht reagierenden, in Wasser bei 20°C zu maximal 0,5 Gew.-% löslichen festen Wirkstoff aus der Gruppe der agrochemischen Wirkstoffe, der Wirkstoffe zur Bekämpfung von Schädlingen im Haushalts- und Hygiene-Bereich und/oder der pharmakologisch wirksamen Stoffe,
- mindestens einen Alkylaryl-polyglykolether der Formel

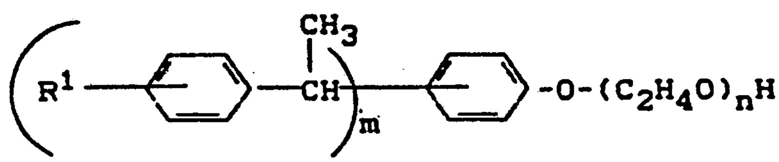

(I)

in welcher

$R^1$, m und n die oben ingegebene Bedeutung haben, im Gemisch mit Alkylaryl-sulfonsäure-Salz der Formel

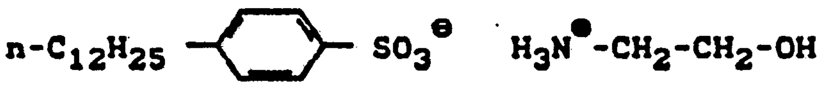

(II)

- Cyclohexanon und Wasser sowie gegebenenfalls Zusatzstoffe

unter Rühren bei einer Temperatur zwischen 10° C und 100°C vermischt und gegebenenfalls anschließend durch Zugabe von wäßriger Säure oder wäßriger Base einen pH-Wert von 2 bis 10 einstellt.

Schließlich wurde gefunden, daß sich die erfindungsgemäßen homogenen wäßrigen Formulierungen, je nich den enthaltenen Wirkstoffen, für verschiedene Zwecke in die Landwirtschaft und im Gartenbau, im Haushalts- und Hygiene-Bereich oder im medizinischen Bereich verwenden lassen.

Es ist als äußerst überraschend zu bezeichnen, daß die erfindungsgemäßen homogenen wäßrigen Formulierungen stabil sind, denn auf Grund des bekannten Standes der Technik war zu erwarten, daß derartige Formulierungen, die keine Verdickungsmittel und auch nur einen geringen Tensidanteil aufweisen, nicht über längere Zeit haltbar sind. So geht aus der DE-A-30 09 944 und der DE-A-30 11 611 bzw. der FR-A-3 452 250 hervor, daß die dort beschriebenen wäßrigen Emulsionen zwingend ein Verdickungsmittel als Stabilisator enthalten. Die in der JP-A 133 635-77 offenbarten Emulsionen und die in der US-A-3 653 075 mitgeteilten, in Wasser dispergierbaren Konzentrate weisen einen im Verhältnis zur Wirkztoffmenge sehr hohen Tenzideanteil auf. Die hervorrigende Beständigkeit der erfindungsgemäßen Formulierungen konnte daher nicht vorausgezehen werden. Überraschend ist außerdem, daß es sich bei den erfindungsgemäßen, homogenen wäßrigen Formulierungen im Gegensatz zu entsprechenden vorbekannten Emulsionen oder Dispereionen um völlig transparente Zubereitungen handelt, die sich optisch nicht von echten Lösungen unterscheiden.

Die erfindungzgemäßen Formulierungen zeichnen sich durch eine Reihe von Vorteilen aus. So erübrigt sich bei ihrer Herstellung ein kostspieliger Zusatz von Verdickungsmitteln oder im Verhältnis zum Wirkstoff großen Mengen an Emulgatoren. Weiterhin sind die erfindungsgemäßen Formulierungen unter den in der Praxis herrschenden Bedingungen stabil.

Bei Langzeit-Lagerungen bleiben diese Formulierungen sowohl bei Temperaturen bis zu 100°C als auch bei tiefen Temperaturen unverändert. Darüber hinaus lassen sich die erfindungsgemäßen Formulierungen in einfacher Weise herstellen. Eine Zwangsdispergierung mit Hilfe von Homogenisatoren ist nicht erforderlich. Die Formulierungen fallen vielmehr direkt als vollkommen transparante Flüssigkeiten an, die auch nach längerer Lagerung bei hohen oder tiefen Temperaturen keine Aufrahmung oder Schichtung (Phasentrennung)

zeigen. Die erfindungzgemäßen Formulierungen sind daher ohne vorheriges Schütteln anwendbar. Schließlich besitzen die erfindungsgemäßen Formulierungen eine niedrige Viskosität, so daß eine genaue Dosierung der jeweile benötigten Menge keine Schwierigkeiten bereitet.

Die erfindungsgemäßen homogenen wäßrigen Formulierungen enthalten mindestens einen nicht hydrolysierenden, mit Cyclohexanon nicht reagierenden, in Wasser bei 30°C zu maximal 0,5 Gew.-% löslichen festen Wirkztoff aus der Gruppe der agrochemischen Wirkstoffe, der Wirkstoffe zur Bekämpfung von Schädlingen im Haushalts- und Hygiene-Bereich und/oder der pharmakologisch wirksamen Stoffe. Diese Wirkstoffe liegen in der Formulierung im flüssigen Zustand vor. Die festen Wirkstoffe müssen in der Formulierung in Cyclohexanon und/oder Emulgator hinreichend löslich sein.

Unter agrochemischen Stoffen sind im vorliegenden Fall alle üblicherweise im Pflanzenschutz verwendbaren festen Wirkstoffe zu verstehen. Hierzu gehören zum Beispiel Insektizide, Akarizide, Nematizide, Fungizide, Herbizide, Wachstumsregulatoren und Düngemittel. Als Beispiele für derartige Wirkstoffe seien im einzelenen genannt:

2-Isopropoxy-phenyl-methylcarbamat
3-Methylthio-4-amino-6-tert.-butyl-1,2,4-triazin-5-on
3-Methylthio-4-isobutylidenamino-6-tert.-butyl-1,2,4-triazin-5-on
2-Chlor-4-ethylamino-6-isopropylamino-1,3,5-triazin
2,3-Dihydro-2,2-dimethyl-7-benzofuranyl-methyl-carbamat
1-(4-Chlorphenoxy)-3,3-dimethyl-1-(1,2,4-triazol-1-yl)-2-butanon
(S)-α-Cyano-3-phenoxybenzyl(1R)-cis-3-(2,2-dibromvinyl) 2,2-dimethylcyclopropancarboxylat
2,2-Dimethyl-3-(ß,ß-dichlorvinyl)-cyclopropancarbonsäure α-cyano-3-phenoxy-4-fluor-benzylester
3-(2,2-Dichlorvinyl)-2,2-dimethyl-cyclopropancarbonsäure-(pentafluorphenyl)-methylester
3-(Phenoxyphenyl)-methyl-(+-)-cis, trans-2,2-dimethyl-3 (2-methyl-1-propenyl)-cyclopropancarboxylat
ß-(4-Chlorphenoxy)-α-(1,1-dimethyl-ethyl)-1H-1,2,4-triazol-1-ithanol
Chrysanthemumsäure-(2,3,4,5-tetrahydrophthalimido)-methylester.

Unter Wirkstoffen zur Bekämpfung von Schädlingen im Haushalts- und Hygiene-Bereich sind im vorliegenden Fall alle üblichen, für diesen Zweck in Frage kommenden festen Wirkstoffe zu verstehen, welche die oben angegebenen Voraussetzungen erfüllen. Als Beispiele für derartige Wirkstoffe seien im einzelnen genannt:

2-Isopropoxy-phenyl-N-methylcarbamat (Cyclohex-1-en-1,2-dicarboximidomethyl)-2,dimethyl-3-(2-methylpropenyl)-cyclopropancarboxylat.

Unter pharmakologisch wirksamen Stoffen sind im vorliegenden Fall vorzugsweise im veterinärmedizinischen Bereich einsetzbare, feste Stoffe zu verstehen, welche die oben angegebenen Voraussetzungen erfüllen. Als Beispiel für derartige Wirkstoffe sei genannt:

2,2-Dimethyl-3-[ß-(p-chlorphenyl)-β-chlorvinyl]-cyclopropancarbonsäure-α-cyano-3-phenoxy-4-fluor-benzylester.

Die in den erfindungsgemäßen homogen, wäßrigen Formulierungen als Emulgatoren enghaltenen Alkylarylpolyglykolether sind durch die Formel (I) allgemein definiert. Die Zahlen für n stellen Durchschnittswerte dar.

Als Beispiele für Alkylaryl-polyglykolether der Formel (I) seien im einzelen genannt:

Bis-[α-methyl-(4-methyl-benzyl)]-phenyl-polyglykolether mit durchschnittlich 27 Oxyethylen-Einheiten pro Molekül.

Bis-[α-methyl-(4-n-dodecyl-benzyl)]-phenyl-polyglykolether mit durchschnittlich 27 Oxyethylen-Einheiten pro Molekül.

Tris-[α-methyl-(methyl-benzyl)]-phenyl-polyglykolether mit durchschnittlich 17 Oxyethylen-Einheiten pro Molekül.

Die in der Praxis verwendeten Emulgatoren dieses Typs sind im allgemeinen Gemische aus mehreren Verbindungen der Formel (I). Insbesondere handelt es sich hierbei um Gemische aus Stoffen der Formel (I), die sich durch den Subititutionsgrad in dem mit der Oxyethylen-Einheit verbundenen Phenylring und durch die Zahl der Oxyethyleneinheiten unterscheiden. Dadurch errichnen sich für den Index m auch gebrochene Zahlen als Mittelwerte. Beispielsweise erwähnt seien Substanzen, für die sich folgende durchschnittliche Zusammensetzungen ergeben:

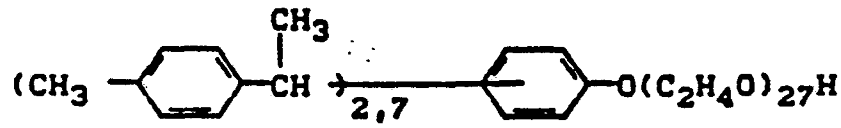

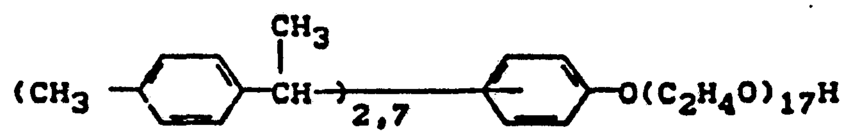

Die Alkylaryl-polyglykolether derr Formel (I) sind bekannt.

Das 4-(n-Dodecyl)-phenyl-sulfonsäure-(3-hydroxyethyl)-ammoniumsalz der Formel (II) ist bekannt. Es wird bei der Herstellung der erfindungsgemäßen Formulierungin ohne zusätzliches Lösungsmittel in Substanz eingesetzt.

Als organisches Lösungsmittel ist in den erfindungsgemäßen Formulierungen Cyclohexanon enthalten.

Als Zusatzstoffe, die in den erfindungsgemäßen Formulierungen enthalten sein können, kommen Dispergiermittel, Kristallisationshemmer, Farbstoffe, Kältestabilisatoren, Synergisten, unpolare orginische Lösungsmittel sowie Säuren und Basen in Betracht.

Als Dispergiermittel können zum Beispiel phosphatierte Alkylphenole, wie phosphatiertes Nonylphenol, eingesetzt werden. Beispiele für Kristallisationshemmer sind Alkylphenole, die pro Mol mit 1 bis 8 Mol Ethylenoxid kondensiert sind. Speziell genannt sei in diesem Zusammenhang Nonylphenol, das pro Mol mit 2 Mol Ethylenoxid kondensiert ist. Als Kristallisationshemmer kommen außerdem auch Magnesium-Salze in Frage: Als Biespiele für Farbstoffe seien Azofarbstoffe und Phthalocyanin-Farbstoffe angeführt. Als Beispiele für Kältistabilisatoren seien Harnstoff, Zucker und Natriumoleat genannt. Als Beispiel für einen Synergisten sei 3,4-Methylendioxy-6-propyl-benzyl-n-butyl-di-ethylen-glykolether (Piperonylbutoxid) genannt.

Unpolare organische Lösungsmittel, die in den erfindungsgemäßen Formulierungen als Zusatzstoffe enthalten sein können, sind vorzugsweise Xylol, Toluol und Dimethylnaphthaline.

Beispiele für Säuren und Basen, die in den erfindungsgemäßen Formulierungen als Zusatzstoffe enthalten sein können, sind starke anorganische oder organische Säuren, wie Phosphorsäure, Zitronensäure und n-Dodecyl-benzolsulfonsäure, und ferner anorganische Basen, wie Natriumhydroxid und Kaliumhydroxid.

In den erfindungsgemäßen homogenen wäßrigen Formulierungen können die prozentualen Anteile der enthaltenen Komponenten innerhalb bestimmter Bereiche variiert werden. Der prozentuale Anteil an Wasser in den erfindungsgemäßen homogenen wäßrigen Formulierungen errechnet sich jeweils als Differenz aus 100 Gew.-% und der Summe der prozentualen Anteile der übrigen Komponenten.

In den erfindungsgemäßen Formulierungen kann das Verhältnis von Wirkstoff(en) einerseits zu Alkylaryl-polyglykolether im Gemisch mit Alkylarylsulfonsäure-Salz andererseits in einem bestimmten Bereich variiert werden.

Bei der Herstellung der erfindungsgemäßen homogenen wäßrigen Formulierungen können vorzugsweise alle diejenigen Komponenten verwendet werden, die bereits im Zusammenhang mit der Beschreibung der erfindungsgemäßen Formulierungen vorzugsweise genannt wurden.

Bei dem erfindungsgemäßen Verfahren können die Wirkstoffe in Substanz oder auch nach vorheriger Lösung in Cyclohexanon und/oder Emulgator eingesetzt werden.

Die Temperatur kann bei dem erfindungsgemäßen Verfahren innerhalb eines größeren Bereiches variiert werden. Im allgemeinen arbeitet man bei einer Temperatur zwischen 10°C und 100°C, vorzugsweise zwischen 30°C und 50°C.

Bei der Durchführung des erfindungsgemäßen Verfahrens geht man im allgemeinen so vor, daß man einen oder mehrere Wirkstoffe im Gemisch mit Cyclohexanon vorlegt, dann unter Rühren mit üblichen Rührgeräten mit Wasser sowie gegebenenfalls mit Zusatzstoffen versetzt und danach unter Rühren gerade soviel Emulgator bzw. Emulgatorgemisch zugibt, das eine homogene klare Flüssigkeit entsteht (= knappe Titration), und gegebenenfalls anschlisßend durch Zugabe von wäßriger Säure oder Base den pH-Wert der Formulierung auf einen Wert zwischen 2 und 10, vorzugsweise zwischen 3,5 und 9 einstellt. Grundsätzlich können die Komponenten in jeder beliebigen Reihenfolge zusammengegeben werden. Letzteres ist insbesondere dann möglich, wenn durch einen Vorversuch ermittelt wurde, in welchem Verhältnis die Komponenten in der Formulierung vorhanden sein müssen, damit eine klare homogene Flüssigkeit entsteht. In vielen Fällen hat es sich als vorteilhaft erwiesen, die frisch hergestellten Formulierungen zu tempern, also für eine Weile auf erhöhte Temperatur zu bringen. Der pH-Wert der Formulierungen muß jeweils auf die enthaltenen Wirkstoffe abgestimmt sein.

Bei den erfindungsgemäßen Formulierungen handelt es sich um vollkommen transparente, wasserklare Flüssigkeiten.

Sie können entweder in der zubereiteten Form oder nach vorheriger Verdünnung appliziert werden. Die Aufwandmenge richtet sich dabei nach der Konzentration der Wirkstoffe in der Formulierung und nach der jeweiligen Indikation.

Die Anwendung der erfindunnsgemäßen Formulierungen erfolgt, gegebenenfalls nach vorheriger Verdünnung, nach den üblichen Methoden, also zum Biespiel durch Spritzen, Sprühen oder gießen.

Die Herstellung der erfindungsgemäßen Formulierungen geht aus den folgenden Beispielen hervor.

**Herstellungsbeispiele**

**Beispiel 1**

1 Gewichtsteil 2-Izopropoxy-phenyl-N-methylcarbamat wird nacheinander mit 12 Gewichtsteilen Cyclohexanon und 81,5 Gewichtsteilen Wasser versetzt. In diese Mischung werden bei Raumtemperatur unter Rühren 5,5 Gewichtsteile eines Emulgatorgemisches gegeben, das zu gleichen Teilen aus den Komponenten besteht, die sich formelmäßen wie folgt beschreiben lassen:

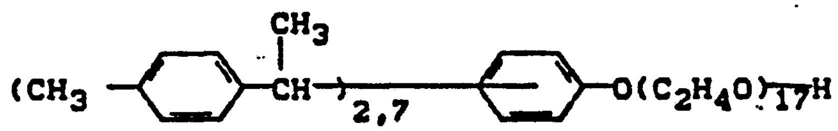 (I-1)

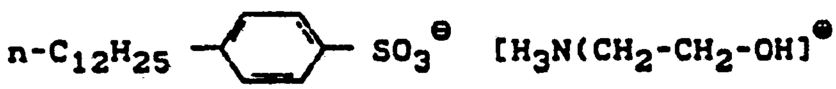 (II)

Nach beendeter Zugabe wird noch 10 Minuten nachgerührt und mittels wäßriger Zitronensäure-Lösung ein pH-Wert von 3,0 eingestellt. Danach heizt man für 10 Minuten auf 65°C auf.

Es bildet sich eine völlig transparente, wasserklare und niedrig viskose Formulierung, die auch nach mehrwöchiger Lagerung bei Temperaturen oberhalb von 50°C keine physikalischen oder chemischen Veränderungen zeigt.

**Beispiel 2**

1 Gewichtsteil 2-Isopropoxy-phenyl-N-methylcarbamat, 0,2 Gewichtsteile (Cyclohex-1-en-1,2-dicarboximidomethyl)-2,2-dimethyl-3-(2-methyl-propenyl)-cyclopropancarboxylat und 1 Gewichtsteil 3,4-Methylendioxy-6-propylbenzyl-n-butyl-di ethylenglykolether werden in 10 Gewichtsteilen Cyclohexanon gelöst und mit 80,8 Gewichtsteilen Wasser versetzt. In diese Mischung werden bei Raumtemperatur unter Rühren 7 Gewichtsteile eines Emulgatorgemisches gegeben, das zu gleichen Teilen aus dem Alkylarylpolyglykolether der Formel (1-1) und 4-n-Dodecyl-phenyl-sulronsäure-(2-hydroxy-ethyl)-ammoniumsalz der Formel (II) besteht. Durch Zugabe wäßriger Zitronensäure-Lösung wird dann ein PH-Wert von 3,5 eingestellt.

Nach beendeter Zugabe wird noch 10 Minuten nachgerührt. Es bildet sich eine völlig transparente, wasserklare und niedrig viskose Formulierung, die auch nach mehrwöchiger Lagerung bei Temperaturen oberhalb von 50°C keine physikalischen oder chemischen Veränderungen zeigt.

**Beispiel 3**

1 Gewichtsteil 2-Isopropoxy-phenyl-H-methylcarbamat wird in 13 Gewichtsteilen Cyclohexanon gelöst und nacheinander mit 0,025 Gewichtsteilen 3-(2,2-Dichlor-vinyl)-2,2-dime-thyl-cyclopropancarbonsäure-(pentafluorphenyl)-methylester und 80,475 Gewichtsteilen Wasser versetzt. In diese Mischung werden bei Raumtemperatur unter Rühren 5,5 Gewichtsteile eines Emulgatorgemisches gegeben, das zu gleichen Teilen aus dem Alkylarylpolyglykolether der Formel (1-1) und 4-n-Dodecyl-phenyl-sulfonsäure-(2-hydroxy-ethyl)-ammoniumsalz der Formel (II) besteht. Durch Zunabe wäßriger Zitronensäure-Lösung wird dann ein pH-Wert von 4,5 eingestellt.

Nach beendeter Zugabe wird noch 10 Minuten nachgerührt. Danach heizt man für 5 Minuten auf 55° C auf. Es bildet sich eine völlig transparente, wasserklare und niedrig viskose Formulierung, die auch nach mehrwöchiger Lagerung bei Temperaturen oberhalb von 50° C keine physikalischen oder chemischen Veränderungen zeigt.

**Beispiel 9**

5 Gewichtsteile β-[4-Chlorphenoxy)-α-(1,1-dimethylethyl)-1H-1,2,4-triazol-1-ethanol werden in 25 Gewichtsteilen Cyclohexanon nelöst und nacheinander mit 9,5 Gewichtsteilen teilen Nonylphenol das pro Mol mit 2 Mol Ethylenoxid kondensiert ist, und 51,9 Gewichteteilen Wasser versetzt. In diese Mischung werden bei Raumtemperatur unter Rühren 8,6 Gewichtsteile eines Emulgatorgemisches gegenen, das zu gleichen Teilen aus dem Alkylarylpolyglykolether der Formel (I-1) und 4-n-Dodecyl-phenyl-sulfonsäure-(2-hy-droxyethyl)-ammoniumsalz der Formel (II) besteht.

Nach beendeter Zugabe wird noch 10 Minuten nachgerührt. Es bildet sich eine völlig transparente, wasserklare und niedrig viskose Formulierung, die auch nach mehrwöchiger Lagerung bei Temperaturen oberhalb von 50° C keine physikalischen oder chemischen Veränderungen zeigt.

**Patentansprüche**

1. Homogene wäßrige Formulierungen, gekennzeichnet durch einen Gehalt von

- 0.1 bis 30 Gew.-% mindestens eines nicht hydrolysierenden, mit Cyclohexanon nicht reagierenden, in Wasser bei 20°C zu maximal 0,5 Gew.-% löslichen festen Wirkstoffes aus der Gruppe der agrochemischen Wirkstoffe, der Wirkstoffe zur Bekämpfung von Schädlingen im Haushalte- und Hygiene-Bereich und/oder der pharmakologisch wirksamen Stoffe,
- 1 bis 15 Gew.-% mindestens eines Alkylaryl-polyglykolethers der Formel

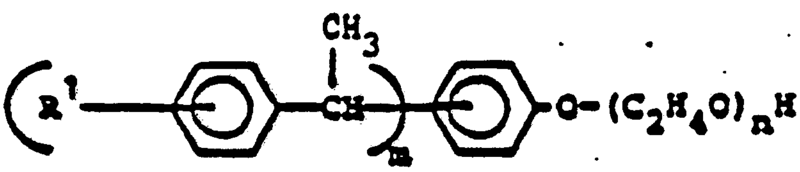

(I)

in welcher
$R^1$ für Alkyl mit 1 bis 12 Kohlenstoffatomen steht,
m für Zahlen von 2 oder 3 steht und
n für Zahlen von 12 bis 40 steht,
im Gemisch mit Alkylarylsulfonsäure-Salz der Formel

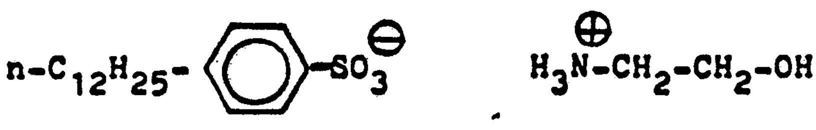

(II)

- 0,5 bis 25 Gew.-% Cyclohexanon
- sowie Wasser und
- gegebenenfalls 0,1 bis 10 Gew.-% an Zusatzstoffen,

wobei die Summe der Komponenten jeweile 100 Gew.-% beträgt.

2. Verfahren zur Herstellung von homogenen wäßrigen Formulierungen gemäß Anspruch 1, dadurch gekennzeichnet, daß man

- mindestens einen nicht hydrolyzierenden, mit Cyclohexanon nicht reagierenden, in Wasser bei 20°C zu maximal 0,5 Gew.-% löslichen resten Wirkstoff aus der Gruppe der agrochemischen Wirkstoffe, der Wirkstoffe zur Bekämpfung von Schädlingen im Haushalts- und Hygiene-Bereich und/oder der pharmakologisch wirksamen Stoffe,
- mindestene einen Alkylaryl-polyglykolether der Formel

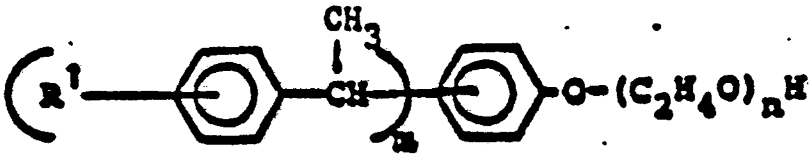

(I)

in welcher
$R^1$ m und n die oben angegebene Bedeutung haben,
im Gemisch mit Alkyleryl-sulfonsäure-Salz der Formel

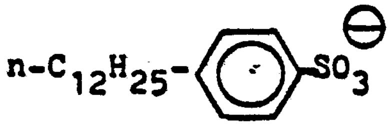

$H_3\overset{\oplus}{N}-CH_2-CH_2-OH$ (II)

- Cyclohexanon und Wasser sowie gegebenenfalls Zusatzstoffe

unter Rühren bei Temperaturen zwischen 10°C und 100°C vermischt und gegebenenfalls anschließend durch Zugabe von wäßriger Säure oder wäßriger Base einen pH-Wert von 2 bis 10 einstellt.

3. Verwendung zur nicht therapeutischen Behandlung von homogenen wäßrigen Formulierungen gemäß Anspruch 1 in der Landwirtschaft und im Gartenbau, im Haushalts- und Hygiene-Bereich und/oder im medizinischen Bereich.

## Claims

1. Homogeneous aqueous formulations characterised in that they contain

- 0.1 to 30% by weight of at least one non-hydrolysing solid active compound which does not reach with cyclohexanone and is soluble in water to an extent of at most 0.5% by weight et 20°C, from the group comprising agrochemical active compounds, active compounds for combating pests in the household and hygiene sectors and/or pharmacologically active compounds,
- 1 to 15% by weight of at least one alkylaryl polyglycol ether of the formula

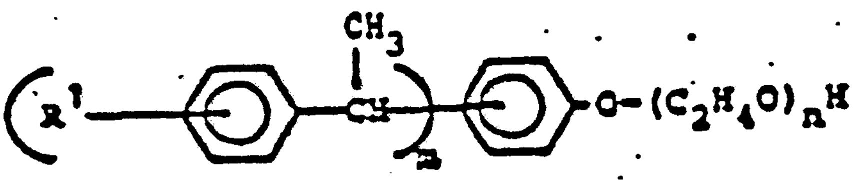

(I)

in which
$R^1$ represents alkyl with 1 to 12 carbon atoms,
m represents numbers of 2 or 3 and
n represents numbers from 12 to 40,
in admixture with an alkylarylsulphonic acid salt of the formula

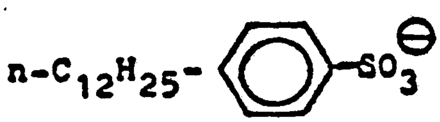

$H_3\overset{\oplus}{N}-CH_2-CH_2-OH$ (II)

- 0.5 to 25% by weight of cyclohexanone,
- and water and
- optionally 0.1 to 10% by weight of additives, the sum of the components being in each case 100% by weight.

2. Process for the preparation of homogeneous aqueous formulations according to Claim 1, characterised in that

- at least one non-hydrolysing solid active compound which does not react with cyclohexanone and is soluble in water to an extent of at most 0.5% by weight at 20°C, from the group comprising agrochemical

active compounds active compounds for combating pests in the household and hygiene sectors and/or pharmacologically active compounds,

- at least one alkylaryl polyglycol ether of the formula

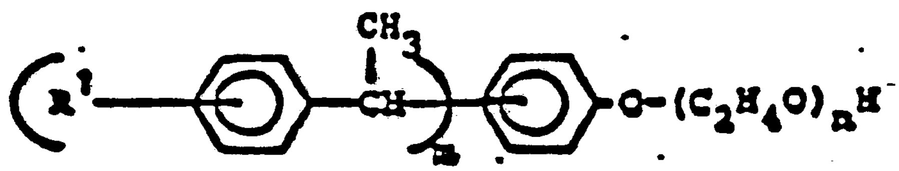

(I)

in which

$R^1$, m and n have the abovementioned meaning, in admixture with an alkylaryl sulphonic acid salt of the formula

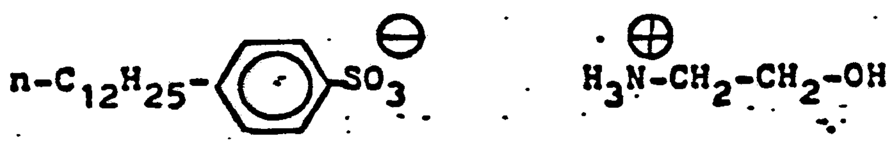

(II)

- cyclohexanone and water and, optionally, additives, are mixed with stirring at temperatures between 10°C and 100°C and, if appropriate, a pH value of 2 to 10 is then adjusted by adding an aqueous acid or an aqueous base.

3. Use, for non-therapeutic treatment, of homogeneous aqueous formulations according to Claim 1 in agriculture and in horticulture, in the household and hygiene sectors and/or in the medical sector.

**Revendications**

Formulations aqueuses homogènes, caractérisées en ce qu'elles contiennent:

0,1 à 30% en poids d'au moins une substance active solide, ne s'hydrolysant pas, ne réagissant pas avec la cyclohexanone, soluble dans l'eau à 20°C à 0,5% en poids au maximum, choisie dans l'ensemble formé par les substances actives agrochimiques, les substances actives destinées à combattre les nuisances et pestes dans le domaine domestique et de l'hygiène et/ou les substances pharmacologiquement actives,

1 à 15% en poids d'au moins un éther polyglycolique d'alkylaryle de formule:

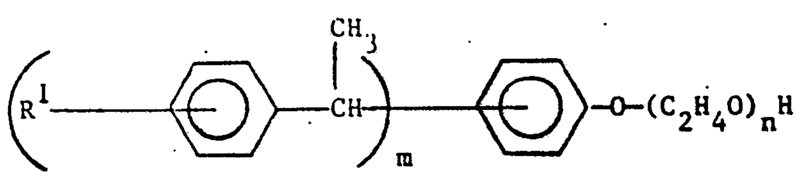

(I)

dans laquelle:

$R^1$ représente un groupe alkyle ayant 1 à 12 atomes de carbone,

m représente les nombres 2 ou 3, et

n représente des nombres valant de 12 à 40,

en mélange avec un sel d'acide alkylaryl-sulfonique de formule:

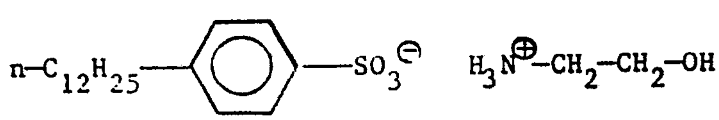

(II)

0,5 à 25% en poids de cyclohexanone, ainsi que de l'eau, et éventuellement 0,1 à 10% d'additifs, la somme des composants formant toujours 100% en poids.

2. Procédé pour préparer des formulations aqueuses homogènes selon la revendication 1, caractérisé en ce que l'on mélange sous agitation, à des températures comprises entre 10°C et 100°C: au moins une substance active solide ne s'hydrolysant pas, ne réagissant pas avec la cyclohexanone, soluble dans l'eau à 20°C à 0,5% en poids au maximum, choisie dans l'ensemble formé par les substances actives agrochimiques, les

substances actives destinées à lutter contre les nuisances et pestes dans le domaine domestique et de l'hygiène et/ou les substances pharmacologiquement actives,

au moins un éther polyglycolique d'alkylaryle de formule:

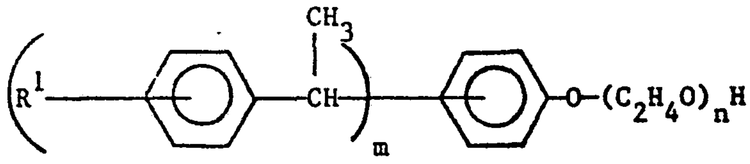

(I)

dans laquelle:

$R^1$, m et n ont le sens précité,

en mélange dans un sel d'acide alkylaryl-sulfonique de formule:

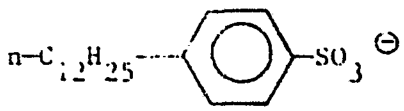

$H_3N^{\oplus}-CH_2-CH_2-OH$ (II)

de la cyclohexanone et de l'eau wt éventuellement des additifs et éventuellement on ajuste ensuite, par addition d'acide aqueux ou de base aqueuse, à une valeur de pH de 2 à 10.

3. Utilisation, pour un traitement non-thérapeutique de formulations aqueuses homogènes selon la revendication 1, en agriculture et en horticulture, dans le domaine doméstique et de l'hygiène et ou dans le domaine médical.